# EUROPÄISCHE PATENTSCHRIFT

(11) **EP 2 959 854 B1**
(45) Veröffentlichungstag und Bekanntmachung des Hinweises auf die Patenterteilung: **21.03.2018**
(21) Anmeldenummer: 14173954.0
(22) Anmeldetag: 25.06.2014
(51) Int. Cl.: A61B 18/14, A61B 18/00

(54) **Chirurgisches Instrument**
Surgical instrument
Instrument chirurgical

(43) Veröffentlichungstag der Anmeldung: 30.12.2015
(73) Patentinhaber: Erbe Elektromedizin GmbH, 72072 Tübingen (DE)
(72) Erfinder: Mayer, Volker, 72072 Tübingen (DE); Brodbeck, Achim, 72555 Metzingen (DE); Weiler, Rolf, 72127 Kusterdingen (DE); Schall, Heiko, 72622 Nürtingen (DE); Amann, Tobias, 72351 Geislingen (DE)
(74) Vertreter: Rüger, Barthelt & Abel

(56) Entgegenhaltungen:
- EP-A2- 1 958 583
- WO-A1-00/47124
- US-A1- 2004 049 185
- US-A1- 2005 113 826
- US-A1- 2005 159 745
- US-A1- 2010 137 854
- US-A1- 2011 257 680

## Beschreibung

Die Erfindung betrifft ein chirurgisches Instrument zur Versiegelung und Trennung von biologischem Gewebe.

Versiegelungsinstrumente der genannten Art sind zur Operation an lebenden menschlichen oder tierischen Patienten in Gebrauch. Sie dienen zur Erfassung und Versiegelung von Gewebe unter Einfluss von Druck und Stromdurchfluss sowie darauf beruhender Wärme. Außerdem können solche Instrumente zum Durchtrennen von koaguliertem bzw. versiegeltem Gewebe dienen.

Die US 2007/0078456 A1 offenbart ein solches Instrument mit zwei beweglichen Branchen, die zum Fassen von Hohlgefäßen dienen. Die beiden Branchen sind bestrombar, so dass das gefasste und zusammengedrückte Hohlgefäß zwischen den Branchen erwärmt und die Gefäßwände miteinander verbunden werden. Das insoweit geschlossene und somit versiegelte Gefäß lässt sich dann in der Versiegelungszone mittels eines verschiebbaren Messers durchtrennen.

Zur Versiegelung des Gefäßes weisen die beiden Branchen des Werkzeugs ebene Kontaktflächen auf, zwischen denen das Gefäß versiegelt wird. Die Kontaktflächen müssen eine erhebliche Mindestbreite aufweisen, um ein sicheres Versiegeln des Gefäßes zu erreichen. Deswegen sind der Miniaturisierung solcher Werkzeuge Grenzen gesetzt.

Weiter ist aus der US 6 113 598 A ein Instrument mit zwei Branchen bekannt, wobei eine (die obere) Branche einen leistenartigen Vorsprung aufweist, der in eine Nut der untern Branche passt, die von zwei federnden Branchenhälften begrenzt wird.

Bei einem solchen Instrument ist eine federnde Klemmung der Gefäßenden während der Koagulation möglich. Dennoch sind auch hier der Miniaturisierung Grenzen gesetzt.

Die US 2002/0115997 A1 offenbart ein Instrument, das insbesondere zur Resektion von Lungengewebe vorgesehen ist. Es weist zwei aufeinander zu und voneinander weg bewegbare Branchen mit Elektrodenstrukturen an beiden Branchen auf. Die Elektrodenstrukturen sind zu beiden Seiten eines Schneidschlitzes, durch den sich ein Trennelement bewegen lässt, so profiliert, dass gefasstes Gewebe formschlüssig gehalten und zwischen den Branchen versiegelt wird. Dabei enthalten sowohl die obere, wie auch die untere Branche auf jeder Seite des Schneidschlitzes jeweils einen positiven und einen negativen Kontakt, um an dem Gewebe einen möglichst breiten Versiegelungssaum zu erzeugen.

Aus der US 8 394 094 ist ein ähnliches elektrochirurgisches Instrument bekannt, bei dem der Schneidelektrode ein federnd gelagertes Widerlager zugeordnet ist.

Aus der US 2004/0049185 A1 ist ein bipolares elektrochirurgisches Instrument zum Schneiden von Gewebe und gleichzeitigem Versiegeln der erzeugten Geweberänder bekannt, das zwei aufeinander zu und voneinander weg bewegliche Branchen aufweist. Beide Branchen weisen jeweils eine mittige Längsnut auf, wobei in einer eine Schneidelektrode und in der anderen ein elastisches Widerlager angeordnet ist. Das Widerlager ist etwas breiter als die ihm zugeordnete Schneidelektrode. Die beiden Nuten sind von Versiegelungselektroden begrenzt. Zwischen den Versiegelungselektroden und der aus Widerlager und Schneidelektrode gebildeten Anordnung sind Gewebeaufnahmeräume ausgebildet.

Es ist Aufgabe der Erfindung, ein Versiegelungsinstrument anzugeben, dessen Konzept eine Miniaturisierung des Werkzeugs gestattet.

Diese Aufgabe wird mit dem Versiegelungsinstrument nach Anspruch 1 und mit einem Verfahren nach Anspruch 15 gelöst:

Das erfindungsgemäße Versiegelungsinstrument für den endoskopischen, laparaskopischen oder offen chirurgischen Anwendungsbereich weist zwei Branchen auf. Die erste Branche weist mindestens zwei Versiegelungselektroden auf, die vorzugsweise miteinander elektrisch verbunden sind. Die zweite Branche weist ebenfalls mindestens zwei Versiegelungselektroden auf, die vorzugsweise miteinander elektrisch verbunden sind. Eine der Branchen (z.B. die obere) weist einen Schneidelektrodenträger mit einem wand- oder schneidenartigen Fortsatz auf. Die andere Branche weist ein entsprechendes Gegenlager für die Schneidelektrode auf. An den Rändern der beiden Branchen sind Versiegelungselektroden angebracht, um Versiegelungssäume am biologischen Gewebe zu erzeugen. Zwischen der Schneidelektrode und den Versiegelungsbacken sind Gewebeaufnahmeräume ausgebildet. Diese weisen vorzugsweise ein solches Volumen auf, dass die dort auftretende Stromdichte deutlich geringer ist, als zwischen den Versiegelungsbacken. Das zwischen Schneidelektrode und den Versiegelungsbacken gefasste, also in den Gewebeaufnahmeräumen befindliche Gewebe wird elektrisch durchströmt, schrumpft dabei aber weniger als das zwischen den Backen zur Ausbildung eines Versiegelungssaums gefasste Gewebe. Das Gewebe bildet wenigstens eine Wulst, die bei geschlossenen Branchen sowohl den ersten Versiegelungsbacken als auch den zweiten Versieglungsbacken annähernd formschlüssig hintergreift. Der Gewebeaufnahmeraum und das in ihm gefasste Gewebe wirken somit als formschlüssige Sicherung für das zwischen den Versiegelungsbacken gefasste Gewebe auch dann, wenn nur ein sehr schmaler Versiegelungssaum und ein sehr filigraner Gewebewulst ausgebildet wird. Das Gewebe kann nicht vor dem Öffnen des Werkzeugs aus dem Versiegelungsinstrument herausrutschen.

Dieses Konzept eröffnet auch die Möglichkeit, mit einer Schneidelektrode zu arbeiten, die zeitgleich mit den Versiegelungselektroden aktiviert wird und somit der Koagulationsprozess und der Schneideprozess zeitgleich beginnt. Das Gewebe kann von der Schneidelektrode durchtrennt werden, bevor die Versiegelung zwischen den Versiegelungsbacken abgeschlossen ist. Die gleichzeitige Einwirkung von Schneidelektrode und Versiegelungsbacken auf das Gewebe ist nicht zwingend, aber möglich. Die zeitliche Abfolge von Schnitt und Versiegelung stellt sich vorzugsweise durch die an der Schneidelektrode und an den Versiegelungsbacken angelegten Spannungen von selbst ein. Es kann dadurch eine Verkürzung der Versiegelungs- und Trennzeit sowie eine Vereinfachung des Arbeitsablaufs und der elektrischen Bestromung des Instruments erreicht werden. Z.B. können eine Versiegelungsspannung (z.B. 97 V) zwischen den Versiegelungsbacken und eine Schneidspannung (z.B. 437 V) zwischen einem Versiegelungsbacken und der Schneidelektrode aus einer Speiseschaltung gleichzeitig angelegt werden. Die Spannungen können aus einem Spartrafo geliefert werden. Die Bestromung kann zunächst mit einem rampenförmigen Stromverlauf und dann mit konstanter Spannung erfolgen. Die Behandlung kann außerdem zeitgesteuert erfolgen. Es kann eine Mindestzeit von 1,4 s und/oder eine Maximalzeit von z.B. 2,8 s festgelegt sein. Als Abschaltkriterium in dem so festgelegten Zeitfenster kann festgelegt sein, dass abgeschaltet wird, wenn nach dem erstem Abfall des Gewebewiderstands ein Wiederanstieg desselben festgestellt wird, wonach gegebenenfalls noch eine zeitliche Zugabe der Bestromung von z.B. 0,4 s erfolgt. Außerdem kann das Instrument oder sein speisendes Gerät mit einer Überspannungs- und Funkenerkennungseinrichtung versehen sein. Zum Löschen erkannter Funken kann eine kurzzeitige Spannungsabsenkung vorgenommen werden.

Außerdem ist das genannte Konzept miniaturisierungsfähig. Durch die formschlüssige Sicherung der Gefäß-oder Gewebeenden in den Gewebeaufnahmeräumen lassen sich die Versiegelungszonen auf sehr schmale, fast linienartige Streifen begrenzen, ohne dass zu befürchten wäre, dass unversiegelte Gewebekanten dem Versiegelungsinstrument vorzeitig entkommen.

Die Gewebeaufnahmeräume, die zu beiden Seiten der Schneidelektrode ausgebildet sind, weisen eine Form auf, wodurch ein besonders guter Formschluss zwischen Gewebe und geschlossenem Instrument erreicht wird. Vorzugsweise ist außerdem die Breite der Gewebeaufnahmeräume größer als die Breite des wandartigen Fortsatzes, der die Schneidelektrode trägt. Außerdem ist die Breite der Gewebeaufnahmeräume vorzugsweise größer als die Breite der Versiegelungsbacken. Auch dies trägt zu einem sicheren Formschluss und zu einer ausreichenden Verminderung der Stromdichte innerhalb des im Aufnahmeraum befindlichen Gewebes bei.

Die Versiegelungsbacken weisen vorzugsweise eine Querschnittskontur mit gerundeten Übergängen auf. Insbesondere sind die Versiegelungsbacken zu den Gewebeaufnahmeräumen hin gerundet, wodurch eine Stromkonzentration vermieden wird. Dabei sind diese Rundungen so gestaltet, dass außer der geringeren Stromkonzentration ein sicherer Formschluss zwischen Gewebe und geschlossenen Branchen möglich ist. Vorzugsweise legen die Versiegelungsbacken der beiden Branchen miteinander Versiegelungsspalte fest, die miteinander einen stumpfen Winkel definieren. Dadurch kann die Rundung der Versiegelungsbacken, die der Schneidelektrode am nächsten liegen, einen großen Rundungsradius erhalten, wodurch gerade hier die Stromdichte begrenzt wird. Ist die Schneideelektrode in der oberen Branche befestigt, können die Rundungsradien der unteren Branche, die in Richtung der Gewebeaufnahmeräume angeordnet sind, einen anderen Wert als die Rundungsradien der oberen Branche umfassen. Vorzugsweise weisen diese Radien jedoch die gleichen Werte auf. Die Rundungsradien der Versiegelungsbacken an der Branchenau-ßenseite, weisen größere Werte auf, als die Rundungsradien in Richtung der Gewebeaufnahmeräume. Dadurch kann der Gewebeeffekt außerhalb der Branchen besser kontrolliert werden. Durch die Schrägstellung der Versiegelungsspalte ergibt sich eine selbsttätige Zentrierung der Branchen zueinander. Außerdem haben die Versiegelungsbacken an ihren Klemmflächen Radien in Richtung der Gewebeaufnahmeräume von z.B. 0,05 mm bis 0,1 mm um einen guten Formschluss zwischen Gewebe und Instrument zu erreichen und Radien an der Branchenaußenseite von z.B. 0,1 mm bis 0,3 mm um gute Versiegelungseigenschaften zu erzielen. Die Klemmflächen legen miteinander einen Versiegelungs- bzw. Klemmspalt von 0 bis 0,1 mm vorzugsweise 0 bis 0,05 mm fest, was ein sicheres Klemmen auch von dünnem Gewebe gestattet.

Die Versiegelungsbacken weisen einen Grundkörper mit isolierenden Bereichen z.B. aus Epoxidharz mit in einer Reihe angeordneten Versiegelungselektroden z.B. aus dem Material des Grundkörpers der Branche, z.B. Edelstahl auf. Der Grundkörper der Branchen kann aus einem Vollmaterial oder einem Stanz- / Biegeteil gebildet sein. Die Elektroden gegenüberliegender Versiegelungsbacken sind vorzugsweise einander nicht überlappend angeordnet bzw. ausgebildet. Die isolierenden Bereiche können durch in die Branchen eingesetzte oder eingeformte (gegossene oder gespritzte) Einlagen oder durch eine Anzahl lokaler Beschichtungen aus Isolationsmaterial gebildet sein. Alternativ kann eine Branche aus Keramik gebildet sein, wobei sie dann im Bereich der Versiegelungselektroden leitend beispielsweise metallisiert ausgebildet ist. Auch ist es möglich, die Branche aus Keramiken mit unterschiedlichen Eigenschaften auszubilden. So kann die Branche beispielsweise ausschließlich aus Keramik gebildet sein, wobei sie dann für die isolierenden Bereiche eine nicht leitende Keramiksubstanz und für den Elektrodenbereich eine leitende Keramiksubstanz aufweist. Die isolierenden Bereiche verhindern einen elektrischen Kurzschluss zwischen den Versiegelungselektroden der beiden Versiegelungsbacken auch dann, wenn sich die Branchen berühren. Der wechselweise Längs-Abstand zwischen Elektroden der beiden Branchen ist 0,1 mm bis 0,3 mm vorzugsweise 0,25 mm. Außerdem ergibt sich in dem biologischen Gewebe ein Stromfluss mit einer Komponente längs der Versiegelungsbacken, d.h. eine Verlängerung der Strompfade mit einer Verbesserung der thermisch biologischen Wirkung. Außerdem können die Versiegelungsbacken an ihren den Gewebeaufnahmeräumen zugewandten Seiten mit nichtmetallischem Material, z.B. PTFE, Kunstharz oder dergleichen versehen sein. Der Stromfluss des Schneidstroms wird so einerseits auf die Schneidelektrode und andererseits auf den zwischen den Versiegelungsbacken gefassten Gewebebereich konzentriert. Die Gefahr des Anklebens des Gewebes in den Gewebeaufnahmeräumen ist beseitigt bzw. deutlich minimiert.

Der Schneidelektrodenträger kann aus Keramik, Kunststoff oder einem isolationsbeschichteten Metall ausgebildet sein. Zusätzlich kann der Schneidelektrodenträger eine Oberfläche aufweisen, an der Gewebe nicht haftet. Dazu kann die Oberfläche des Schneidelektrodenträger antihaftend ausgebildet beispielsweise beschichtet sein. Zusätzlich weist der Schneidelektrodenträger eine hohe Kriechstromfestigkeit beziehungsweise einen hohen CTI (Comparative Tracking Index) Wert, vorzugsweise größer 600 auf. Die Schneidelektrode ist vorzugsweise als dünne Leiste ausgeführt, die eine freiliegende Stirnfläche aufweist trägt und über geeignete Mittel, beispielsweise mehrere Füße, in oder an dem Elektrodenträger verankert ist. Auf diese Weise wird die thermische Trägheit der Schneidelektrode auf ein Minimum begrenzt. Das Gegenlager kann federnd angeordnet sein, um ein Zerquetschen des Gewebes zu verhindern und den Schnitt durch elektrische Einwirkung zu führen. Dies kommt der Versiegelungssicherheit zugute, da auf diese Art und Weise, eine Bewegung aufgrund des Gewebeschneidens, zwischen Schneidelektrode der einen und Gegenlager der anderen Branche, nicht in gleichem Ausmaß eine Branchenbewegung und damit eine Bewegung zwischen gegenüberliegenden Versiegelungsbacken bewirkt. Alternativ oder zusätzlich kann der Schneidelektrodenträger federnd gelagert sein. In diesem Fall kann das Gegenlager starr befestigt sein.

Ist der Schneidelektrodenträger aus Kunststoff ausgebildet, wird die Schneidelektrode vorzugsweise mit Kunststoff umspritzt. Die Schneidelektrode kann z.B. aus Edelstahlblech bestehen. Die Breite der Schneidelektrode beträgt vorzugsweise etwa 0,1 mm. Die Dicke des Kunststoffs zu beiden Seiten der Schneidelektrode beträgt vorzugsweise etwa 0,15 mm. Die Schneidelektrode schließt vorzugsweise mit den beiden, ihre Flanken bedeckenden Kunststoffwänden bündig ab (kein Schneidenüberstand). Als Kunststoff wird vorzugsweise ein Duroplast eingesetzt. Alternativ kann die Schneidelektrode im ungenutzten Zustand die Kunststoffwände geringfügig, beispielsweise um 0,02 mm bis 0,04 mm überragen. Der Überstand kann sich während des Betreibens des Instrumentes, beispielsweise durch Abbrand der Schneidelektrodenträgers, verändern.

Ist der Schneidelektrodenträger aus Keramik, vorzugsweise ZrO₂-Keramik gefertigt, werden der Schneidelektrodenträger und die Schneidelektrode getrennt vorgefertigt und dann zusammengefügt. Die Breite der Schneidelektrode beträgt vorzugsweise 0,2 bis 0,25 mm. Sie kann aus einem Blech, insbesondere Edelstahlblech bestehen. Die isolierenden Wände zu beiden Seiten der Schneidelektrode haben vorzugsweise eine Dicke von 0,15 mm. Vorzugsweise ist ein Schneidenüberstand von 0,02 mm bis 0,04 mm festgelegt. Die Schneidelektrode kann mit dem Schneidelektrodenträger formschlüssig verbunden, verklemmt oder stoffschlüssig verklebt werden. Typischerweise existiert ein Spalt zwischen der Schneidelektrode und den dünnen isolierenden Wänden des Schneidelektrodenträgers. Dieser Spalt kann mit Klebstoff, Silikon oder ähnlichem gefüllt werden. Alternativ kann die Schneidelektrode eine seitliche Isolation aufweisen, z.B. aus einem Lack oder einer Beschichtung z.B. Parylene. Dadurch wird die seitliche Kontaktfläche der Schneidelektrode isoliert und der Einfluss eines im Spalt zwischen Schneidelektrode und Schneidelektrodenträger befindlichen Fluid auf die Schneidwirkung der Schneidelektrode minimiert vorzugsweise verhindert.

Das der Schneidelektrode gegenüberliegend angeordnete Gegenlager ist vorzugsweise federnd gelagert. Der Federhub kann auf weniger als 1mm, vorzugsweise 0,5 mm festgelegt sein. Das Gegenlager dient der Aufrechterhaltung der Klemmkraft zwischen den Versiegelungsbacken. Erreicht wird dies durch die mechanische Entkopplung der Gewebeklemmung im Bereich der Versiegelungsbacken von der Gewebeklemmung zwischen Schneidelektrode und Gegenlager. Damit werden auch die Schrumpfungen des Gewebes beim Schnitt und beim Versiegeln von der Klemmkraft zwischen den Versiegelungsbacken entkoppelt. Diese Wirkung tritt auch bei Ausführungsformen mit starr angeordnetem Gegenlager und federnd gelagerter Schneidelektrode auf.

Die Oberseite bzw. Gewebeauflagefläche des Gegenlagers sitzt bei geschlossenen Branchen auf einer anderen Ebene als die Versiegelungsbacken. Die Schneideebene der Schneidelektrode steht über die Versiegelungsbacken der an derselben Brache angeordneten Versiegelungselektroden hinaus. Damit findet der Schneidprozess auf einer anderen Ebene statt als der Versiegelungsprozess.

Im geöffneten Zustand der Branchen kann das Gegenlager die Versiegelungsbacken derselben Branche überragen. Beim Schließen der Branchen wird dann das federelastische oder federelastisch gelagerte Gegenlager komprimiert und dabei in die Branche gedrückt. Dadurch entsteht die Federkraft, die dem Schneidelement entgegenwirkt.

Das Gegenlager besteht vorzugsweise aus einem Isolator mit Antihaftoberfläche, z.B. aus PTFE, und ähnlichen oder gleichen Kriechstromeigenschaften wie der Schneidelektrodenträger. Die federnde Lagerung des Gegenlagers ergibt ein Schnittverhalten, bei dem sich der Schneidspalt an die Gewebeschrumpfung beim Schnitt anpasst. Das längliche Gegenlager kann an seinem proximalen Ende unabhängig von seinem distalen Ende federn und so mit der Schneidelektrode auch einen keilförmigen Spalt festlegen, wenn längs der Schneidelektrode unterschiedliche Gewebedicken vorliegen.

Es ist alternativ möglich, das Schneidelement federnd zu lagern. Das Gegenlager kann in diesem Fall starr oder ebenfalls federnd beweglich gelagert werden. Es ist auch möglich, eines der Elemente um eine Querachse schwenkbar und das jeweils andere Element federnd zu lagern, um z.B. bei besonders kleinem Federhub von z.B. weniger als 0,5 mm eine gute Anpassung der Schneidelektrode und des Gegenlagers an unterschiedliche Gewebestärken entlang der Schneidelektrode zu erreichen.

Das Verfahren zur Versiegelung und Trennung von Gewebe umfasst vorzugsweise mindestens die folgenden Schritte:
Zwischen zwei Branchen wird Gewebe so gefasst, dass es zwischen Versiegelungsbacken sowie zwischen der Schneidelektrode und der Gewebeauflagefläche geklemmt wird, wobei zwischen den geschlossenen Versiegelungsbacken und einem Schneidelektrodenträger Gewebeaufnahmeräume ausgebildet sind, die wenigstens teilweise von Teilen des Gewebes gefüllt werden, und die Energiezufuhr zu den Versiegelungsbacken und der Schneidelektrode findet gleichzeitig statt.

Durch die gleichzeitige Energiezufuhr an die Versiegelungselektroden und der Schneidelektrode finden die Prozesse Gefäße versiegeln und Gefäße trennen (schneiden) gleichzeitig statt. Das Trennen von Gefäßen kann beendet sein, bevor das Versiegeln der Gefäße endet. Die Gewebeaufnahmeräume sichern während des Prozesses der Gefäßversiegelung die Gewebesäume innerhalb der Branchen bis zur vollständigen Beendigung des Versiegelungsprozesses. Durch die erfindungsgemäße Ausbildung der Branchen in Verbindung mit der erfindungsgemäßen Art und Weise der Energiezufuhr kann das Bearbeiten, insbesondere das Trennen und das Versiegeln von Gefäßen in höchster Qualität erfolgen. Der gesamte Bearbeitungsvorgang ist wegen des zeitgleichen Starts von Versiegelung und Schneiden insgesamt kurz und dauert im Allgemeinen nicht länger als die Versiegelung für sich alleine.

Weitere Einzelheiten vorteilhafter Ausführungsformen der Erfindung ergeben sich aus der Zeichnung der Beschreibung oder Ansprüchen. Es zeigen:
Figur 1 ein Versiegelungsinstrument in schematisierter perspektivischer Gesamtdarstellung;
Figur 2 das Werkzeug des Versiegelungsinstruments nach Figur 1 in vergrößerter perspektivischer teilweise geschnittener Darstellung;
Figur 3 das Werkzeug nach Figur 2 in geschlossenem Zustand in Querschnittsdarstellung;
Figur 4 das Werkzeug nach Figur 3 beim Versiegeln und Trennen eines Hohlgefäßes;
Figur 5 die Schneidelektrode des Werkzeugs nach Figur 2 in einer perspektivischen teilweise geschnittenen Darstellung in einer ersten Ausführungsform;
Figur 6 die Schneidelektrode nach Figur 5 in Querschnittsdarstellung;
Figur 7 eine abgewandelte Ausführungsform einer Schneidelektrode und eines Schneidelektrodenträgers in Längsschnittdarstellung; und
Figur 8 eine weitere abgewandelte Ausführungsform einer Schneidelektrode in perspektivischer Darstellung.

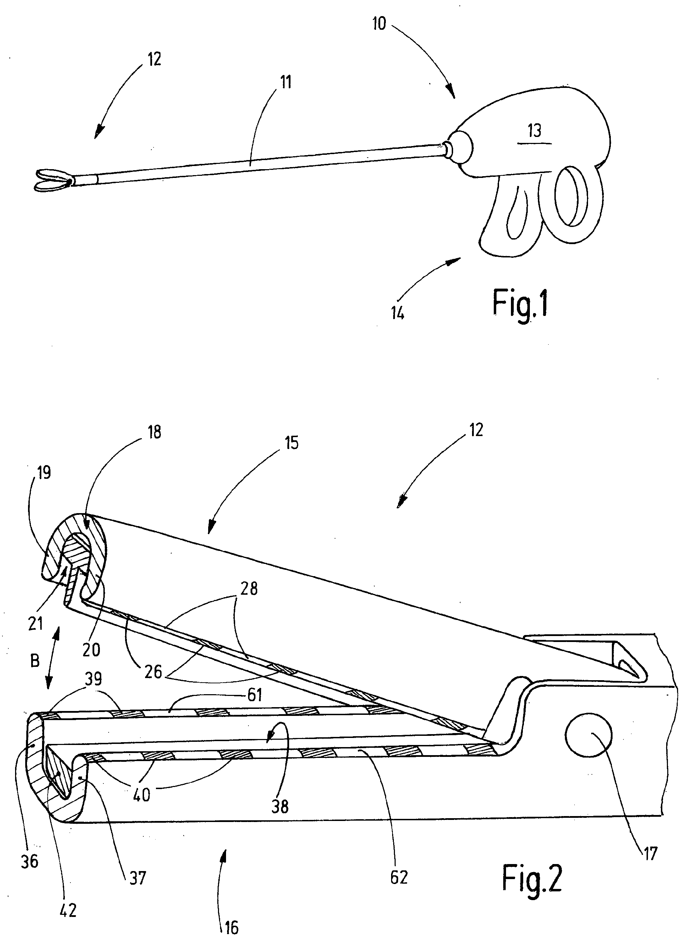

In Figur 1 ist ein Instrument 10 mit einem länglichen Schaft 11 veranschaulicht, der an seinem distalen Ende ein Werkzeug 12 trägt. Das proximale Ende des Schafts 11 ist mit einem Gehäuse 13 verbunden, an dem Betätigungselemente 14 zur Bewegung und Betätigung des Werkzeugs 12 angeordnet sind. Das Instrument 10 ist ein Versiegelungsinstrument. Entsprechend dient das Werkzeug 12 zum Versiegeln und gegebenenfalls Durchtrennen von Gewebe, wobei im Gewebe enthaltene Gefäße und Lumen an dem entstehenden Gewebesaum geschlossen und somit abgedichtet sein sollen.

Das an dem Instrument 10 vorgesehene Werkzeug 12 ist auf besondere Weise ausgebildet. Es weist eine erste Branche 15, in Figur 2 die obere Branche, und eine zweite Branche 16, in Figur 2 die untere Branche, auf, von denen mindestens eine um eine Schwenkachse 17 schwenkbar gelagert ist. Dabei kann je nach Anwendungsfall die erste Branche 15 oder die zweite Branche 16 oder es können beide Branchen 15, 16 um eine gemeinsame oder um unterschiedliche Schwenkachsen schwenkbar oder anderweitig beweglich gelagert sein, so dass die Branchen 15, 16 aufeinander zu und voneinander weg bewegt werden können.

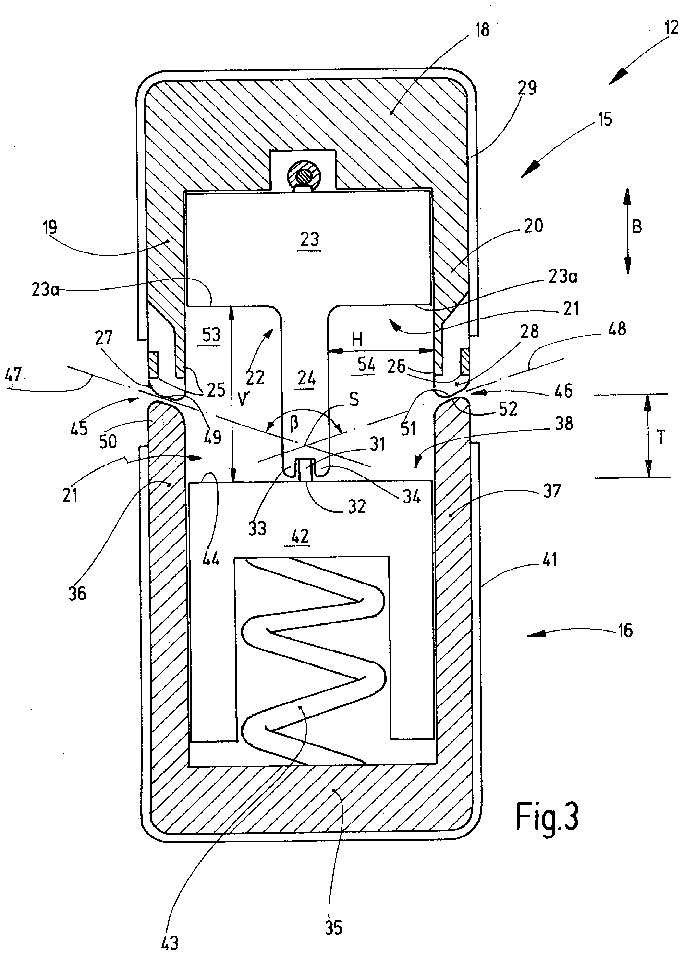

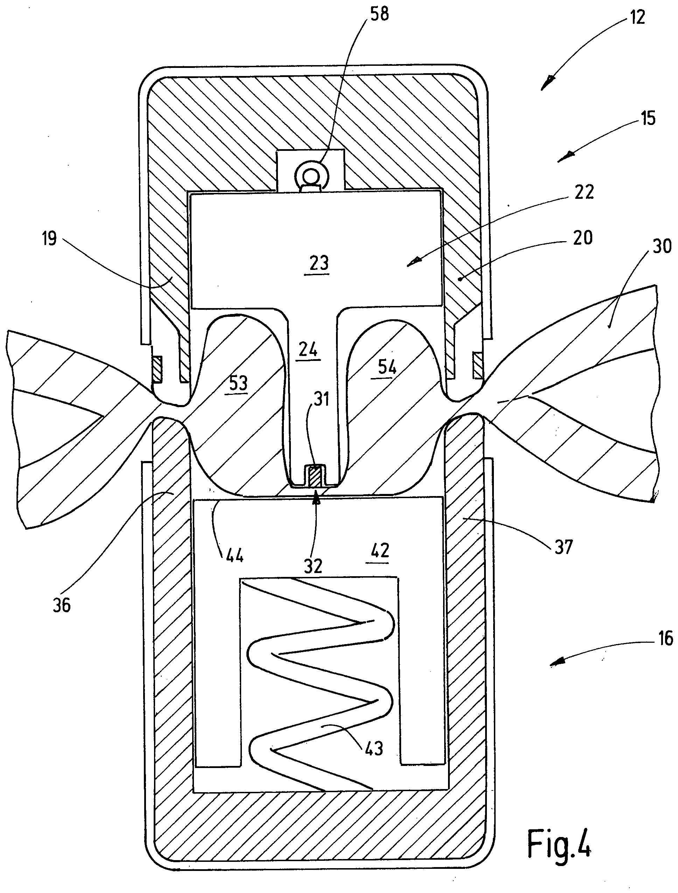

Die Branchen 15, 16 sind in den Figuren 3 und 4 nochmals gesondert im Querschnitt veranschaulicht. Die erste Branche 15 kann beispielsweise aus Metall oder einem sonstigen biegefesten Material bestehen. Sie weist einen Grundkörper 18 mit U-profilförmigem Querschnitt auf. Der Grundkörper 18 weist zwei zueinander parallele erste Versiegelungsbacken 19, 20, die elektrisch vorzugsweise miteinander verbunden sind auf, und die zwischen einander eine Nut 21 begrenzen. Diese erstreckt sich vorzugsweise über den größten Teil der Länge der ersten Branche 15 und dient der Aufnahme eines Schneidelektrodenträgers 22. Dieser weist einen Fuß 23 auf, der der Kontur der Nut 21 angepasst ist und ortsfest, unbeweglich in dieser sitzt. Von dem im Querschnitt beispielsweise rechteckigen Fuß 23 erstreckt sich, ausgehend von einer Fußfläche 23a, vorzugsweise mittig zwischen den beiden Versiegelungsbacken 19, 20 ein wandartiger Fortsatz 24 weg, so dass dieser aus der Nut 21 herausragt. Die Nut 21 ist von den Rändern der Versiegelungsbacken 19, 20 begrenzt, an denen Versiegelungselektroden 25, 26 ausgebildet sind. Die Fußfläche 23a ist gegenüber den Versiegelungselektroden 25, 26 zurückversetzt angeordnet. Die Versiegelungselektroden 25, 26 können elektrisch leitend mit dem Grundkörper 18 verbunden sein. Die Versiegelungselektroden 25, 26 bilden, wie insbesondere aus Figur 2 hervorgeht, vorzugsweise eine Reihe voneinander beabstandeter leitender Einzelflächen, die durch isolierende Bereiche 27, 28 voneinander getrennt sind. Die Branche 15 kann außerdem einen elektrisch isolierenden Überzug 29 aufweisen, so dass in die Branche 15 eingeleiteter elektrischer Strom nur an den Versiegelungselektroden 25, 26 in biologisches Gewebe 30 (Figur 4) eingeleitet werden kann. Zwischen dem isolierenden Überzug 29 und den isolierenden Bereichen 27, 28 besteht vorzugsweise ein Abstand, sodass das Material der Versiegelungselektroden in diesem Bereich nicht isoliert ist. Dadurch wird eine gute Versiegelungsqualität erreicht.

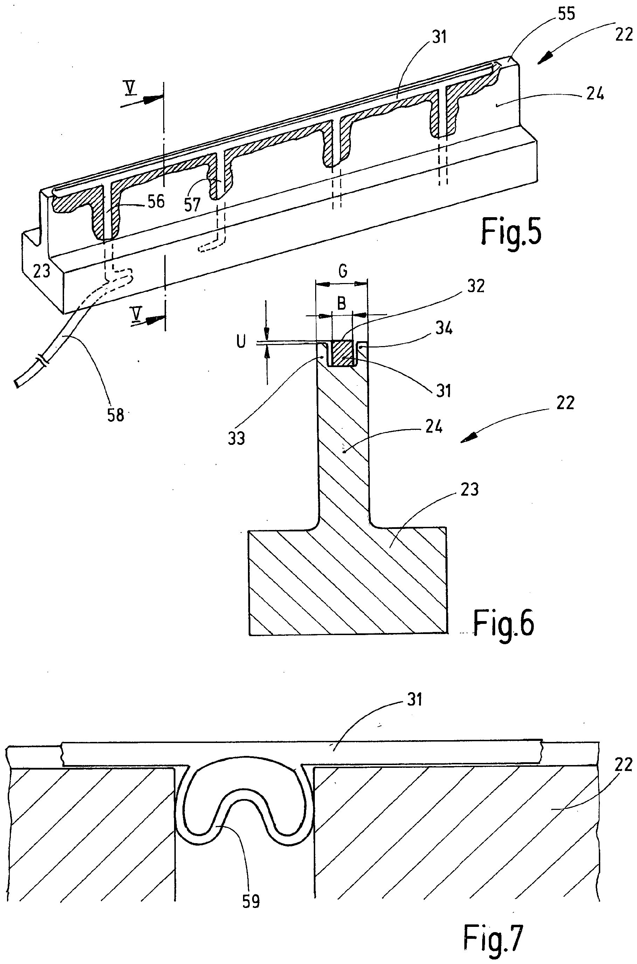

Der Schneidelektrodenträger 22 weist eine Schneidelektrode 31 auf, die an seiner Stirnseite angeordnet ist. Die Schneidelektrode 31 sitzt dabei in einer Nut oder Ausnehmung des wandartigen Fortsatzes 24, wobei die Schneidelektrode 31 mit einer Stirnfläche 32 frei liegt. Die Schneidelektrode 31 ist zwischen zwei Nutwänden 33, 34 gefasst. Die Nutwände 33, 34 weisen vorzugsweise eine Breite auf, die etwa so groß ist wie die Breite der Schneidelektrode 31. Es wird dazu auf Figur 6 Bezug genommen. Die Breite B der Schneidelektrode 31 kann im Bereich von 0,05 bis 0,25 mm liegen und beträgt vorzugsweise 0,1 mm. Die isolierenden Nutwände 33, 34 weisen vorzugsweise eine Dicke der gleichen Größenordnung auf. Beispielsweise haben sie eine Dicke von 0,15 mm. Sie schließen bis auf einen geringen Überstand miteinander ab. Vorzugsweise beträgt der Überstand U der Stirnfläche 32 der Schneidelektrode 31 über die Stirnflächen der Nutwände 33, 34 lediglich wenige 10 µm, beispielsweise 0 bis 40 µm.

Die Länge des wandartigen Fortsatzes 24, gemessen von dem Fuß 23 zu der Schneidelektrode 31 hin, ist vorzugsweise so ausgeführt, dass die Stirnfläche 32 die Versiegelungsbacken 19, 20 um etwa 0,5 bis 1 mm, vorzugsweise 0,9 mm überragt. Entsprechend wird die Gewebeauflagefläche 44 der zweiten Branche 16 durch den wandartigen Fortsatz 24 der ersten Branche 15 im geschlossenen Zustand der Branchen, in dem sich die Versiegelungsbacken 19, 36 und 20, 37 berühren bzw. nahezu berühren, etwa um denselben Abstand in die zweiten Branche 16 hineingedrückt. Dieses Maß ist in Figur 3 rechts neben dem Werkzeug 12 als Maß T angegeben.

Aus Figur 3 weiter ersichtlich ist die zweite Branche 16, die ebenfalls einen Grundkörper 35 vorzugsweise aus elektrisch leitendem Material aufweist. Der Grundkörper 35 weist wiederum einen U-förmigen Querschnitt auf, bei dem zwischen zwei zweiten Versiegelungsbacken 36, 37 eine Nut 38 ausgebildet ist. An den elektrisch vorzugsweise miteinander verbundenen zweiten Versiegelungsbacken 36, 37 der zweiten Branche 16 sind wieder Versiegelungselektroden 39, 40 ausgebildet (Figur 2), die in einer Reihe entlang der oberen Kanten der Versiegelungsbacken 36, 37 angeordnet sind. Die Versiegelungselektroden 39, 40 sind in Backenlängsrichtung gemessen kürzer als die isolierenden Bereiche 27, 28 der ersten Branche 15. Gleichermaßen sind die Versiegelungselektroden 25, 26 der ersten Branche 15 in Backenlängsrichtung gemessen, kürzer als die isolierenden Bereiche 61, 62, also die Abstände zwischen den zweiten Versiegelungselektroden 39, 40 der zweiten Branche 16. Außerdem sind die ersten Versiegelungselektroden 25, 26 an der ersten Branche 15 so positioniert, dass sie beim Schließen der Branchen 15, 16 zwischen die zweiten Versiegelungselektroden 39, 40, d.h. auf die isolierenden Bereiche 61, 62 der zweiten Branche 16 treffen. Auf diese Weise ist ein elektrischer Kurzschluss zwischen der ersten Branche 15 und der zweiten Branche 16 nicht möglich.

Die zweite Branche 16 kann, wie Figur 3 zeigt, wiederum mit einem isolierenden Überzug 41 versehen sein. In der Nut 38 ist ein Gegenlager 42 für die Schneidelektrode 31 angeordnet. Das Gegenlager 42 kann parallel zu den Versiegelungsbacken 36, 37 beweglich gelagert sein und beispielsweise gegen die Kraft eines Federelements 43 in die Nut 38 hineindrückbar sein. Das Gegenlager 42 ist beispielsweise ein aus Kunststoff oder Keramik oder einem sonstigen elektrischen Nichtleiter bestehendes leistenartiges Element mit ebener Gewebeauflagefläche 44, die eine Anlagefläche für das biologische Gewebe 30 bildet und in Ruheposition unterhalb der Versiegelungselektroden 39, 40, d.h. innerhalb der Nut 38 steht. Bedarfsweise kann die Gewebeauflagefläche 44 auch strukturiert oder in Kurvenform oder eine Kombination dieser ausgebildet sein.

Das Federelement 43 kann eine Druckfeder sein, die sich an einem Ende an dem Boden der Nut 38 abstützt und am anderen Ende mit dem Gegenlager 42 in Wirkverbindung steht. Es können auch mehrere solcher Druckfedern längs der Branche 16 in einer Reihe angeordnet sein. Alternativ kann als Feder an dem Nutboden ein Elastomerelement anliegen oder befestigt sein, auf dem sich das Gegenlager 42 abstützt. Der federnde Hub des Gegenlagers 42 kann relativ gering sein und beispielsweise auf weniger als einen Millimeter, vorzugsweise 0,5 mm begrenzt sein. Der federnde Hub des Gegenlagers ist abhängig vom Federelement 43. Bei einem vorgespannten Federelement 43 reduziert sich dieser Hub auf beispielsweise 0,5 mm oder weniger, bei einem nicht vorgespannten Federelement 43 umfasst dieser Hub auch den Weg, mit dem die Vorspannung erzeugt wird und kann 1 mm oder größer sein. Alternativ können die Funktionen des Gegenlagers 42 und des Federelements 43 in einem Bauteil realisiert werden, beispielsweise ein Bauteil hergestellt aus Elastomer.

Die Versiegelungsbacken 36, 37 sind an ihrer Oberseite gerundet ausgebildet. Sie begrenzen mit den Versiegelungsbacken 19, 20 der ersten Branche 15 jeweils paarweise einen Spalt 45, 46 dessen Orientierung in Figur 3 durch strichpunktierte Linien 47, 48 angedeutet ist. Die Orientierung der Spalte 45, 46 kann durch an den Versiegelungsbacken 19, 20, 36, 37 vorgesehene ebene Facetten 49, 50, 51, 52 bestimmt sein. Die von den Spalten 45, 46 festgelegten durch die Linien 47, 48 definierten Richtungen bilden einen stumpfen Winkel mit einem Scheitel bzw. Schnittpunkt S, der die Spitze des Winkels markiert. Diese weist zu der Schneidelektrode 31 hin. Vorzugsweise liegt der stumpfe Winkel in einem Bereich von 130 bis 150°.

Zur funktionsbestimmenden Geometrie des Werkzeugs 12 gehören außerdem zwei Gewebeaufnahmeräume 53, 54. Diese sind zu beiden Seiten des wandartigen Fortsatzes 24 ausgebildet und umfassen Bereiche der Nuten 21 und 38. Sie werden vertikal zwischen dem Fuß 23 und der Gewebeauflagefläche 44 des Gegenlagers 42 begrenzt. Diese Vertikalausdehnung V ist in Figur 3 eingetragen und liegt bei geschlossenen Branchen ohne Gewebe zum Beispiel im Bereich von 0,7 mm bis 2,5 mm, vorzugsweise 1,4 mm. Der Spalt 45, 46 ist etwa mittig in Bezug auf die Vertikalausdehnung V des Gewebeaufnahmeraums 53 bzw. 54 oder vorzugsweise leicht zu der Fußfläche 23a hin versetzt angeordnet.

Die beiden Gewebeaufnahmeräume 53, 54 sind vorzugsweise gleich groß ausgebildet. Sie weisen eine Horizontalausdehnung H auf, die durch den Abstand zwischen dem wandartigen Fortsatz 24 und den jeweiligen Versiegelungsbacken 19, 36 bzw. 20, 37 bestimmt ist. Diese Horizontalausdehnung H ist vorzugsweise deutlich größer als die in gleicher Richtung zu messende Dicke des Fortsatzes 24. Vorzugsweise beträgt die Horizontalausdehnung eines dieser Gewebeaufnahmeräume etwa das 0,2 bis 0,6-fache der Vertikalausdehnung.

Das insoweit beschriebene Instrument arbeitet wie folgt:
Zur Versiegelung und Trennung von Geweben insbesondere Hohlgefäßen oder Hohlgefäße enthaltenden Geweben wird dieses zwischen den Branchen 15, 16 gefasst. Durch entsprechende Betätigung der Betätigungselemente 14 werden die Branchen 15, 16 so aufeinander zu bewegt, dass biologisches Gewebe 30 gemäß Figur 4 gefasst wird. Das Gewebe 30 wird dabei zwischen den Versieglungsbacken 19, 36 und 20, 37 einem Druck ausgesetzt während es in den Gewebeaufnahmeräumen 53, 54 relativ entspannt liegt. Die Schneidelektrode 31 übt jedoch auch einen großen Druck auf das Gewebe 30 aus.

Zur Gewebeversieglung wird zwischen den Branchen 15, 16 eine elektrische Spannung vorzugsweise eine hochfrequente Wechselspannung wirksam, so dass zwischen den Elektroden 25, 26 der ersten Branche 15 und den Elektroden 39, 40 der zweiten Branchen 16 ein elektrischer Strom durch das biologische Gewebe 30 fließt, um dieses zu erwärmen und eine Fusion des gefassten Gewebes herbeizuführen. Zeitgleich wird die Schneidelektrode 31 aktiviert. Dieser wird auch eine elektrische Spannung vorzugsweise eine HF-Spannung zugeführt, deren Bezugspotenzial auf einer der Branchen 15, 16 vorzugsweise auf der zweiten Branche 16, das heißt den Versiegelungselektroden 39, 40 liegt.

Aufgrund der geringen Fläche der Schneidelektrode von vorzugsweise etwa 1 mm² bis 3 mm² ist die Stromdichte an der Stirnfläche 32 so hoch, dass das biologische Gewebe 30 zügig durchtrennt wird. Das unter der Schneidelektrode 31 schrumpfende Gewebe ist durch das federnde Gegenlager 42 unterstützt. Es wird von der Gewebeauflagefläche 44 zu der Stirnfläche 32 hin gedrückt, so dass es ständig in Kontakt mit der Schneidelektrode 31 bleibt. Die Stromdichte nimmt jedoch schon in geringem Abstand von der Stirnfläche 32 drastisch ab. Der für den Stromfluss zur Verfügung stehende Querschnitt wechselt an der Seitenwand des Fortsatzes 24 von dem engen Spaltquerschnitt auf den weiten Querschnitt des Gewebeaufnahmeraums 53 bzw. 54. Das dort vorhandene biologische Gewebe wird deswegen nur schwach erwärmt und schrumpft deutlich weniger, als das Gewebe unterhalb der Stirnfläche 32. Auf diese Weise bilden sich zwischen den Versiegelungsbacken 19, 36 und den Versiegelungsbacken 20, 37 jeweils Versiegelungssäume aus, in denen alle Lumen in dem Gewebe 30 geschlossen sind und eine schweißnahtartige Verbindung in dem Gewebe ausgebildet ist. Gleichzeitig oder zeitversetzt wird das Gewebe unterhalb der Schneidelektrode 31 durchtrennt. Solange die Branchen 15, 16 geschlossen sind verhindern die in den Gewebeaufnahmeräumen 53, 54 sitzenden Gewebewülste ein Entkommen des Gewebes 30 aus dem Werkzeug 12. Erst wenn dieses geöffnet wird, werden die Gewebesäume freigegeben und der Behandlungsprozess ist beendet.

Eine wesentliche Aufgabe bei der Behandlung des Gewebes 30 kommt der Schneidelektrode 31 und dem Schneidelektrodenträger 22 zu. Um einerseits die Gewebeaufnahmeräume 53, 54 bei möglichst geringer Gesamtbreite des Werkzeugs 12 möglichst groß zu gestalten ist die Gesamtbreite G (Figur 6) des wandartigen Fortsatzes 24 möglichst gering. Vorzugsweise ist sie wenige zehntel Millimeter groß. Dies hat auch den Effekt, dass die Stromdichte nur unterhalb der Schneidelektrode 31 einen für die Gewebedurchtrennung ausreichenden Wert hat und dann zur Ausbildung der in den Gewebeaufnahmeräumen 53, 54 sitzenden Wülste sprungartig abfällt.

Figur 5 zeigt die Variante eines Schneidelektrodenträgers 22 aus Keramik. Der wandartige Fortsatz 24 weist an seiner Stirnseite eine Nut 55 auf, in der die leistenartige Schneidelektrode 31 sitzt. Diese weist vorzugsweise mehrere Befestigungsfortsätze 56, 57 auf, die sich durch Öffnungen des Schneidelektrodenträgers 22 erstrecken. Die so gebildete Elektrodenanordnung kann aus einem mit Keramik ausgebildeten Schneidelektrodenträger 22 verklebt sein. An dem Fuß 23 können ein oder mehrere Befestigungsfortsätze 56, 57 mit einer elektrischen Leitung 58 verbunden sein, die der Stromzuführung zu der Schneidelektrode 31 dient.

Der Schneidelektrodenträger 22 bewirkt unabhängig von seiner konkreten Ausbildung eine Potenzialtrennung zwischen der Schneidelektrode 31 und den Versiegelungselektroden 25, 26, 39, 40. Während die Versiegelungselektroden 39 und 40 beispielsweise als Neutralelektroden dienen, können die Versieglungselektroden 25, 26 mit einer die Versiegelung bewirkenden Spannung beaufschlagt werden, die vorzugsweise geringer ist als die zum Durchtrennen des Gewebes vorgesehene, der Schneidelektrode 31 zugeleitete Spannung. Die Versiegelungselektroden 25, 26, 39, 40 weisen vorzugsweise eine Gesamtelektrodenfläche auf, die größer als das zehnfache, vorzugsweise größer als das zwanzigfache der Fläche der Stirnfläche 32 ist, so dass die Energiezufuhr nur an der Stirnfläche 32 einen Schnitt, an den Versiegelungselektroden 25, 26, 39, 40 jedoch keine Durchtrennung des Gewebes bewirkt.

Figur 7 veranschaulicht eine abgewandelte Ausführungsform des Schneidelektrodenträgers 22. Die Schneidelektrode 31 sitzt wiederum in einer Nut des Schneidelektrodenträgers und ist dort mit federnden Füßen 59 verankert. Der Grundkörper des Schneidelektrodenkörpers 22 kann aus Keramik oder Kunststoff bestehen. Als Kunststoff eignet sich ein Duroplast oder ein Silikon, der insbesondere hochtemperaturstabil, flammhemmend und stabil gegen Funkenerosion ist, sowie ein CTI größer 600 hat. Außerdem sollte er eine ausreichend hohe mechanische Festigkeit und eine Wärmeleitfähigkeit größer ein W/mK aufweisen.

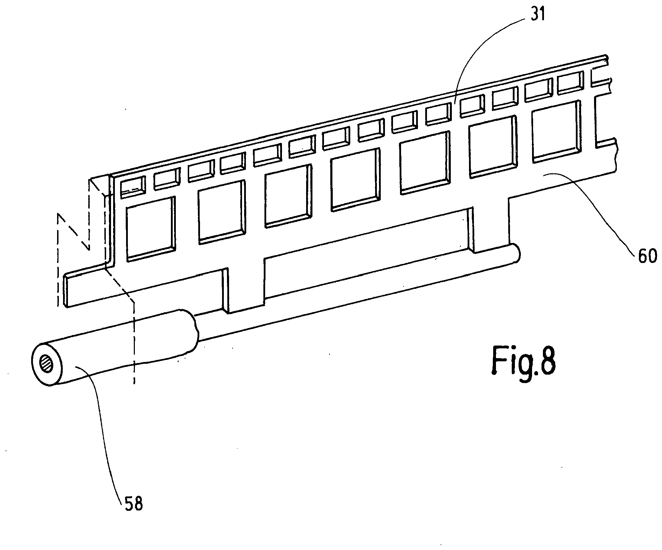

Ein solcher Kunststoff kann insbesondere mit einem Elektrodeninlay 60 nach Figur 8 versehen sein. Die Schneidelektrode 31 kann auch hier wie bei allen anderen Ausführungsformen aus einem Blech, z.B. aus Edelstahl, bestehen. Durch die in dem Elektrodeninlay 60 vorhandenen zahlreichen Fenster wird eine innige Verbindung von Kunststoff und Elektrodeninlay 60 sowie ein geringer Wärmeeintrag in den Kunststoff erreicht.

Ein insbesondere für filigrane Arbeiten vorgesehenes chirurgisches Instrument 10 weist zwei Branchen 15, 16 mit Versiegelungsbacken 19, 20, 36, 37 auf, die mit einer Versiegelungsspannung vorzugsweise zwischen 80 und 120 Volt beaufschlagbar sind. Zwischen den Branchen 15, 16 sind Gewebeaufnahmeräume 53, 54 definiert, die von einem wandartigen Fortsatz 24 voneinander getrennt sind, der an seiner unteren Kante eine Schneidelektrode 31 trägt. Zwischen einer Branche 16 und der Schneidelektrode liegt eine Schneidspannung von 300 bis 500 Volt an. Beim Schließen der Branchen wird das gefasste Gewebe gleichzeitig zwischen den Versiegelungsbacken 19, 36 sowie 20, 37 versiegelt und von der Schneidelektrode 31 durchtrennt. Selbst wenn das Gewebe beispielsweise nach 0,3 bis 0,5 Sekunden vollständig durchtrennt ist und die Versiegelung weitere Zeit, insgesamt etwa 1,5 bis 3,5 Sekunden benötigt, kann eine Gefäßtrennung mit geschlossenen, versiegelten Gefäßenden erzielt werden. Dabei wirken die Gewebeaufnahmeräume 53, 54 als formschlüssige Sicherung der bereits getrennten Gefäßenden und stellen somit die Ausbildung geschlossener Gefäßenden mit Gewebesäumen in dem Werkzeug 12 sicher. Die Gefäßenden sind im Vergleich zu der Versiegelungszone schwächer oder nicht koaguliert und bilden somit wulstartige Verdickungen, die verhindern, dass die Gewebesäume bei geschlossenem Instrument aus diesem entkommen.

**Bezugszeichenliste:**

| | |
|---|---|
| 10 | Instrument |
| 11 | Schaft |
| 12 | Werkzeug |
| 13 | Gehäuse |
| 14 | Betätigungselemente |
| 15 | Erste Branche |
| 16 | Zweite Branche |
| 17 | Schwenkachse |
| 18 | Grundkörper der ersten Branche 15 |
| 19, 20 | Erste Versiegelungsbacke |
| 21 | Nut |
| 22 | Schneidelektrodenträger |
| 23 | Fuß des Schneidelektrodenträgers |
| 23a | Fußfläche |
| 24 | Wandartiger Fortsatz des Schneidelektrodenträgers |
| 25, 26 | Versiegelungselektroden der ersten Branche |
| 27, 28 | Isolierende Bereiche von 15 |
| 29 | Elektrisch isolierender Überzug |
| 30 | Biologisches Gewebe |
| 31 | Schneidelektrode |
| 32 | Stirnfläche |
| 33, 34 | Nutwände |
| B | Breite der Schneidelektrode |
| U | Überstand |
| 35 | Grundkörper |
| 36, 37 | Zweite Versiegelungsbacke |
| 38 | Nut |
| 39, 40 | Versiegelungselektroden der zweiten Branche |
| 41 | Isolierender Überzug |
| 42 | Gegenlager |
| 43 | Federelement |
| 44 | Gewebeauflagefläche |
| 45 | Spalt zwischen den Versiegelungsbacken 19 und 36 |
| 46 | Spalt zwischen den Versiegelungsbacken 20 und 37 |
| 47 | Linie zur Veranschaulichung der Orientierung des Spalts 45 |
| 48 | Linie zur Veranschaulichung der Orientierung des Spalts 46 |
| 49 - 52 | Facetten |
| 53, 54 | Gewebeaufnahmeräume |
| V | Vertikalausdehnung der Gewebeaufnahmeräume 53, 54 |
| H | Horizontalausdehnung der Gewebeaufnahmeräume 53, 54 |
| 55 | Nut |
| 56, 57 | Befestigungsfortsätze |
| T | Überstand Schneidelektrode über Versiegelungselektrode |
| 58 | Leitung |
| 59 | Füße |
| 60 | Elektrodeninlay |
| 61,62 | Isolierende Bereiche von 16 |

## Patentansprüche

1. Chirurgisches Instrument (10):
mit einer ersten Branche (15), die zwei in einem Abstand zueinander angeordnete erste Versiegelungsbacken (19, 20) aufweist, die zwischen einander eine erste Nut (21) begrenzen,
mit einem elektrisch isolierenden Schneidelektrodenträger (22), der einen in der Nut (21) angeordneten Fuß (23) mit einer Fußfläche (23a) aufweist, die gegen die an den ersten Versiegelungsbacken (19, 20) angeordneten Versiegelungselektroden (25, 26) zurückgesetzt ist und von der sich im Abstand zu den ersten Versiegelungsbacken (19, 20) der ersten Branche (15) ein wandartiger Fortsatz (24) weg erstreckt,
mit einer Schneidelektrode (31), die in den Schneidelektrodenträger (22) mit einer Stirnfläche (32) freiliegend eingebettet ist,
mit einer zweiten Branche (16), die zwei in einem Abstand zueinander angeordnete zweite Versiegelungsbacken (36, 37) aufweist, die zwischen einander eine zweite Nut (38) begrenzen,
wobei die Branchen (15, 16) in einer Bewegungsrichtung (B) aufeinander zu und voneinander weg beweglich sind, um geschlossen und geöffnet zu werden,
mit einem in der zweiten Nut (38) angeordneten Gegenlager (42) für die Schneidelektrode (31), wobei das Gegenlager (42) eine Gewebeauflagefläche (44) aufweist, die im geschlossenen Zustand der Branchen (15, 16) gegen die Versiegelungselektroden 39, 40 der zweiten Versiegelungsbacken (36, 37) zurück versetzt ist und
eine Anlagefläche für das biologische Gewebe (30) bildet,
wobei bei geschlossenen Branchen (15, 16) in Bewegungsrichtung (B) zwischen der Gewebeauflagefläche (44) des Gegenlagers (42) und der Fußfläche (23a) des Fußes (23) sowie quer zu der Bewegungsrichtung (B) zwischen dem Fortsatz (24) und den ersten Versiegelungsbacken (19, 36) sowie zwischen dem Fortsatz (24) und den zweiten Versiegelungsbacken (20, 37) Gewebeaufnahmeräume (53, 54) ausgebildet sind, die vertikal zwischen dem Fuß (23) und der Gewebeauflagefläche (44) des Gegenlagers (42) begrenzt sind und deren Horizontalausdehnung (H) durch den Abstand zwischen dem wandartigen Fortsatz (24) und den Versiegelungsbacken (19,36; 20,37) bestimmt ist,wobei die Versiegelungsbacken (19, 36; 20, 37) paarweise je einen Versiegelungsspalt (45, 46) festlegen und
wobei die ersten und die zweiten Versiegelungsbacken (19,36; 20,37) zu den Gewebeaufnahmeräumen (53, 54) hin gerundet sind.

2. Versiegelungsinstrument nach Anspruch 1, **dadurch gekennzeichnet, dass** die Gewebeaufnahmeräume (53, 54) eine quer zu der Bewegungsrichtung (B) zwischen dem Fortsatz (24) und den Versiegelungsbacken (19, 36; 20, 37) zu messende Breite (H) aufweisen, die geringer ist, als die in Bewegungsrichtung (B) zwischen dem Gegenlager (42) und dem Fuß (23) zu messende Höhe.

3. Versiegelungsinstrument nach einem der vorigen Ansprüche, **dadurch gekennzeichnet, dass** die Gewebeaufnahmeräume (53, 54) eine quer zu der Bewegungsrichtung (B) zwischen dem Fortsatz (24) und den Versiegelungsbacken (19, 36; 20, 37) zu messende Breite (H) aufweisen, die größer ist als die in gleicher Richtung zu messende Dicke des wandartigen Fortsatzes (24).

4. Versiegelungsinstrument nach einem der vorigen Ansprüche, **dadurch gekennzeichnet, dass** die Versiegelungsbacken (19, 36; 20, 37) eine Querschnittskontur mit gerundeten Übergängen aufweisen.

5. Versiegelungsinstrument nach Anspruch 1, **dadurch gekennzeichnet, dass** beide von je zwei Versiegelungsbacken (19, 36; 20, 37) paarweise gebildete Versiegelungsspalte (45, 46) miteinander einen stumpfen Winkel (ß) festlegen.

6. Versiegelungsinstrument nach Anspruch 5, **dadurch gekennzeichnet, dass** der Winkel (ß) einen Scheitel (S) aufweist, der zu der Schneidelektrode hinweist.

7. Versiegelungsinstrument nach einem der vorigen Ansprüche, **dadurch gekennzeichnet, dass** jeder Versiegelungsbacken (19, 36; 20, 37) wenigstens einen isolierenden Bereich (27, 28, 61, 62) aufweist, der zwischen in einer Reihe angeordneten Versiegelungselektroden (25, 26, 39, 40) liegt.

8. Versiegelungsinstrument nach Anspruch 7, **dadurch gekennzeichnet, dass** die Versiegelungselektroden (25, 26; 39, 40) eines Versiegelungsbackens (19, 20; 36, 37) elektrisch miteinander verbunden sind.

9. Versiegelungsinstrument nach Anspruch 7, **dadurch gekennzeichnet, dass** die Versiegelungselektroden (25, 26; 39, 40) einander paarweise gegenüberliegender Versiegelungsbacken (19, 36;20, 37) einander nicht überlappend angeordnet sind.

10. Versiegelungsinstrument nach einem der vorigen Ansprüche, **dadurch gekennzeichnet, dass** der Schneidelektrodenträger (22) aus Keramik oder Kunststoff ausgebildet ist.

11. Versiegelungsinstrument nach einem der vorigen Ansprüche, **dadurch gekennzeichnet, dass** die Schneidelektrode (31) eine durchgehende, die freiliegende Stirnfläche (32) tragende Leiste aufweist, die über mehrere Befestigungsfortsätze (56, 57) in dem Elektrodenträger (22) verankert ist.

12. Versiegelungsinstrument nach einem der vorigen Ansprüche, **dadurch gekennzeichnet, dass** die Schneidelektrode (31) eine durchgehende, die freiliegende Stirnfläche (32) tragende Leiste aufweist, die über mehrere federnde Füße (59) in dem Elektrodenträger (22) verankert ist.

13. Versiegelungsinstrument nach einem der vorigen Ansprüche, **dadurch gekennzeichnet, dass** der Schneidelektrodenträger (22) in der ersten Nut (21) starr und das Gegenlager (42) in der zweiten Nut (38) beweglich angeordnet ist oder dass der Schneidelektrodenträger (22) in der ersten Nut (21) beweglich und das Gegenlager (42) in der zweiten Nut (38) starr oder beweglich angeordnet ist.

## Claims

1. Surgical instrument (10):
with a first branch (15) which has two first sealing jaws (19, 20) arranged at a distance from each other and delimiting between them a first groove (21),
with an electrically insulating cutting electrode carrier (22) which has a foot (23) arranged in the groove (21) and having a foot surface (23 a), which is set back relative to the sealing electrodes (25, 26) arranged on the first sealing jaws (19, 20) and from which a wall-like extension (24) extends away at a distance from the first sealing jaws (19, 20) of the first branch (15),
with a cutting electrode (31) which is embedded in the cutting electrode carrier (22) with an exposed end face (32),
with a second branch (16) which has two second sealing jaws (36, 37) arranged at a distance from each other and delimiting between them a second groove (38),
wherein the branches (15, 16) are movable towards each other and away from each other in a movement direction (B), in order to be closed and opened,
with a counter-bearing (42) for the cutting electrode (31) arranged in the second groove (38), wherein the counter-bearing (42) has a tissue-support surface (44) which, in the closed state of the branches (15, 16), is set back relative to the sealing electrodes (39, 40) of the second sealing jaws (36, 37) and forms a contact surface for the biological tissue (30),
wherein when the branches (15, 16) are closed, in the movement direction (B) between the tissue-support surface (44) of the counter-bearing (42) and the foot surface (23a) of the foot (23), and transversely to the movement direction (B) between the extension (24) and the first sealing jaws (19, 36), and between the extension (24) and the second sealing jaws (20, 37), tissue-receiving spaces (53, 54) are formed which are delimited vertically between the foot (23) and the tissue-support surface (44) of the counter-bearing (42), and the horizontal extension (H) of which is determined by the distance between the wall-like extension (24) and the sealing jaws (19, 36; 20, 37), wherein the sealing jaws (19, 36; 20, 37) in pairs establish a respective sealing gap (45, 46), and
wherein the first and the second sealing jaws (19, 26; 20, 37) are rounded towards the tissue-receiving spaces (53, 54).

2. Sealing instrument according to claim 1, **characterised in that** the tissue-receiving spaces (53, 54) have a width (H) measured transversely to the movement direction (B) between the extension (24) and the sealing jaws (19, 36; 20, 37) which is smaller than the height measured in the movement direction (B) between the counter-bearing (42) and the foot (23).

3. Sealing instrument according to any of the preceding claims, **characterised in that** the tissue-receiving spaces (53, 54) have a width (H) measured transversely to the movement direction (B) between the extension (24) and the sealing jaws (19, 36; 20, 37) which is greater than the thickness of the wall-like extension (24) measured in the same direction.

4. Sealing instrument according to any of the preceding claims, **characterised in that** the sealing jaws (19, 36; 20, 37) have a cross-sectional contour with rounded transitions.

5. Sealing instrument according to claim 1, **characterised in that** the two sealing gaps (45, 46), which are formed in pairs each by two sealing jaws (19, 36; 20, 37), establish an obtuse angle (p) between them.

6. Sealing instrument according to claim 5, **characterised in that** the angle (β) has an apex (S) which points towards the cutting electrode.

7. Sealing instrument according to any preceding claims, **characterised in that** each sealing jaw (19, 36; 20, 37) has at least one insulating region (27, 28, 61, 62) which lies between sealing electrodes (25, 26, 39, 40) arranged in a row.

8. Sealing instrument according to claim 7, **characterised in that** the sealing electrodes (25, 26; 39, 40) of a sealing jaw (19, 20; 36, 37) are connected together electrically.

9. Sealing instrument according to claim 7, **characterised in that** the sealing electrodes (25, 26; 39, 40) of sealing jaws (19, 36; 20, 37) lying opposite each other in pairs are arranged not overlapping each other.

10. Sealing instrument according to any of the preceding claims, **characterised in that** the cutting electrode carrier (22) is made of ceramic or plastic.

11. Sealing instrument according to any of the preceding claims, **characterised in that** the cutting electrode (31) has a continuous strip which carries the exposed end face (32) and is anchored in the electrode carrier (22) via several fixing extensions (56, 57).

12. Sealing instrument according to any of the preceding claims, **characterised in that** the cutting electrode (31) has a continuous strip which carries the exposed end face (32) and is anchored in the electrode carrier (22) via several sprung feet (59).

13. Sealing instrument according to any of the preceding claims, **characterised in that** the cutting electrode carrier (22) is arranged rigidly in the first groove (21) and the counter-bearing (42) is arranged movably in the second groove (38), or that the cutting electrode carrier (22) is arranged movably in the first groove (21) and the counter-bearing (42) is arranged rigidly or movably in the second groove (38).

## Revendications

1. Instrument chirurgical (10) :
comprenant un premier mors (15) qui présente deux premières joues de scellement (19, 20) qui sont disposées à distance l'une de l'autre et délimitent une première rainure (21) entre elles,
comprenant un support d'électrode de coupe (22) électriquement isolant qui présente une embase (23) disposée dans la rainure (21) et dotée d'une surface d'embase (23a) qui est en retrait par rapport aux électrodes de scellement (25, 26) disposées sur les premières joues de scellement (19, 20) et à partir de laquelle s'étend un prolongement (24) en forme de paroi, à distance des premières joues de scellement (19, 20) du premier mors (15),
comprenant une électrode de coupe (31) qui est encastrée dans le support d'électrode de coupe (22), en laissant une face frontale (32) dégagée,
comprenant un deuxième mors (16) qui présente deux deuxièmes joues de scellement (36,37) qui sont disposées à distance l'une de l'autre et délimitent une deuxième rainure (38) entre elles,
les mors (15, 16) pouvant être rapprochés et éloignés l'un de l'autre dans une direction de déplacement (B) pour pouvoir être fermés et ouverts,
comprenant une butée (42) pour l'électrode de coupe (31), qui est disposée dans la deuxième rainure (38), la butée (42) présentant une surface d'appui de tissu (44) qui, à l'état fermé des mors (15, 16) est en retrait par rapport aux électrodes de scellement (39, 40) des deuxièmes joues de scellement (36, 37) et forme une surface de contact pour le tissu biologique (30),
sachant que lorsque les mors (15, 16) sont fermés, des espaces de réception de tissu (53, 54) sont formés, dans la direction de déplacement (B), entre la surface d'appui de tissu (44) de la butée (42) et la surface d'embase (23a) de l'embase (23), ainsi que perpendiculairement à la direction de déplacement (B), entre le prolongement (24) et les premières joues de scellement (19, 36) et entre le prolongement (24) et les deuxièmes joues de scellement (20, 37), espaces qui sont délimités verticalement entre l'embase (23) et la surface d'appui de tissu (44) de la butée (42) et dont la dimension horizontale (H) est déterminée par la distance entre le prolongement (24) en forme de paroi et les joues de scellement (19, 36 ; 20, 37), les joues de scellement (19, 36 ; 20, 37) définissant par paires respectivement un interstice de scellement (45, 46), et les premières et les deuxièmes joues de scellement (19, 36 ; 20, 37) étant arrondies côté espaces de réception de tissu (53, 54).

2. Instrument de scellement selon la revendication 1, **caractérisé en ce que** les espaces de réception de tissu (53, 54) présentent une largeur (H) à mesurer perpendiculairement à la direction de déplacement (B), entre le prolongement (24) et les joues de scellement (19, 36 ; 20, 37), qui est inférieure à la hauteur à mesurer dans la direction de déplacement (B), entre la butée (42) et l'embase (23).

3. Instrument de scellement selon une des revendications précédentes, **caractérisé en ce que** les espaces de réception de tissu (53, 54) présentent une largeur (H) à mesurer perpendiculairement à la direction de déplacement (B), entre le prolongement (24) et les joues de scellement (19, 36 ; 20, 37), qui est supérieure à l'épaisseur du prolongement (24) en forme de paroi à mesurer dans la même direction.

4. Instrument de scellement selon une des revendications précédentes, **caractérisé en ce que** les joues de scellement (19, 36 ; 20, 37) présentent un contour de section transversale avec des transitions arrondies.

5. Instrument de scellement selon la revendication 1, **caractérisé en ce que** les deux interstices de scellement (45, 46) formés respectivement par paires par deux joues de scellement (19, 36 ; 20, 37) définissent un angle (β) obtus entre eux.

6. Instrument de scellement selon la revendication 5, **caractérisé en ce que** l'angle (β) présente un sommet (S) qui est orienté en direction de l'électrode de coupe.

7. Instrument de scellement selon une des revendications précédentes, **caractérisé en ce que** chaque joue de scellement (19, 36 ; 20, 37) présente au moins une zone isolante (27, 28, 61, 62) qui se situe entre des électrodes de scellement (25, 26, 39, 40) disposées en une rangée.

8. Instrument de scellement selon la revendication 7, **caractérisé en ce que** les électrodes de scellement (25, 26 ; 39, 40) d'une joue de scellement (19, 20 ; 36, 37) sont reliées électriquement entre elles.

9. Instrument de scellement selon la revendication 7, **caractérisé en ce que** les électrodes de scellement (25, 26 ; 39, 40) de joues de scellement (19, 36 ; 20, 37) se faisant face par paires sont disposées de manière à ne pas se chevaucher mutuellement.

10. Instrument de scellement selon une des revendications précédentes, **caractérisé en ce que** le support d'électrode de coupe (22) est réalisé en céramique ou en matière plastique.

11. Instrument de scellement selon une des revendications précédentes, **caractérisé en ce que** l'électrode de coupe (31) présente une baguette continue qui porte la face frontale (32) dégagée et est ancrée dans le support d'électrode (22) par l'intermédiaire de plusieurs pattes de fixation (56, 57).

12. Instrument de scellement selon une des revendications précédentes, **caractérisé en ce que** l'électrode de coupe (31) présente une baguette continue qui porte la face frontale (32) dégagée et est ancrée dans le support d'électrode (22) par l'intermédiaire de plusieurs pieds (59).

13. Instrument de scellement selon une des revendications précédentes, **caractérisé en ce que** le support d'électrode de coupe (22) est disposé de façon fixe dans la première rainure (21) et la butée (42) est disposée de façon mobile dans la deuxième rainure (38), ou que le support d'électrode de coupe (22) est disposé de façon mobile dans la première rainure (21) et la butée (42) est disposée de façon fixe ou mobile dans la deuxième rainure (38)
